# EUROPEAN PATENT APPLICATION

(11) **EP 4 431 942 A1**
(43) Date of publication of application: **18.09.2024**
(21) Application number: 23161900.8
(22) Date of filing: 14.03.2023
(51) Int. Cl.: G01N 33/18, G01N 1/14

(54) **PRE-SETTLING AND SAMPLING SYSTEM AND METHODS FOR SAMPLE EXTRACTION FOR ANALYSIS**

(71) Applicant: Aquamonitrix Limited, Carlow R93 N529 (IE)
(72) Inventor: STAUNTON, Alan, Tullow, Co. Carlow, R93 N529 (IE); SUNJKA, Peter John, Tullow, Co. Carlow, R93 N529 (IE); RAJIAH, Prakash, Tullow, Co. Carlow, R93 N529 (IE)
(74) Representative: Murgitroyd & Company

(57) **Abstract**

The present invention relates to a pre-settling, sample extraction, and sample analysis system (100) for extracting a sample from a mixed liquor contained in an aeration tank (400) for automated sample analysis. The system comprises a settling vessel (130) configured to receive a fluid sample from the aeration tank, wherein the fluid sample in settled in the settling vessel for a predetermined settling time period, and a sample extraction device (150) the sample extraction device configured to receive a portion of the fluid sample contained in the settling vessel (130); an analyser (200) arranged inline to, and coupled to the system (100); a control unit (170) configured to control a fluid from the aeration tank to the settling vessel (120) and extraction of the sample using the sample extraction device and delivery to the analyser. Related method and arrangements of a sample extraction device are also described.

## Description

### Field

The present invention relates to a sample extraction device and systems and methods for sample extraction and provision for analysis.

### Background

Current methods of sample analysis for Activated Sludge Process, ASP, applications are challenging due to the nature of the sample matrix involved. For example, mixed fluid within an aeration tank, or aeration basin, has a relatively high content of suspended solids, for example typical levels of Mixed Liquor Suspended Solids, MLSS, would be in the range of 3,000 to 10,000 milligrams per litre (3,000 to 10,000 mg/L). The ASP treatment stage also involves aeration for the introduction of oxygen to the process, so the matrix tends to be quite turbulent with gas bubbles present. In one approach a system utilizing filter elements may be used for sampling the mixed fluid however such arrangements are often prone to being clogged by material from the sample matrix and accordingly may require multiple instances of maintenance, down time, and replacement parts, leading to high ongoing operational costs due to down time and need for replacement parts during the course of use and operation.

Accordingly, there is a requirement to provide a system, device and a method to enable sampling of a mixed fluid within an aeration tank having lower operational costs, less downtime and maintenance requirements as well as reduce need for replacement parts.

### Summary

The aim of the present invention is to provide an improved or alternative method, apparatus and system for extracting, by a sample extraction device configured for coupling to a settling vessel, a supernatant from a mixed liquor sample for analysis. The analysis may comprise automated online and/or in-situ fluid or water sample analysis. The system and method are configured to address the issues and problems associated with previous approaches.

According to a first aspect there is provided a sample extraction device (150) configured for use in extracting a sample S from a fluid, for provision to an analyser (200) for analysis, the sample extraction device:
a first inner tube (153) having an upper end (159-1) and a lower end (158-1), the lower end defining an opening and the upper end defining a closed end of the inner tube (153);
a second outer tube (155) having an upper end (159-2) and a lower end (158-2), the lower end defining an opening, the outer tube comprising an overflow outlet (151);
wherein the outer tube portion (155) is arranged generally parallel to the inner tube portion (153), and wherein the lower end (158-2) of the outer tube (155) is in fluid communication with the lower end (158-1) of the inner tube (153);
an air bleed port (160) located at the upper end (159-1) of the inner tube portion (153);
a sample inlet (156) for extracting a sample for transfer to the analyser located in the inner tube between the upper and lower ends thereof;
the sample extraction device (150) configured to be coupled to a settling vessel (130) such that in use it is in fluid communication with the settling vessel at the lower ends of the inner and outer tube portions thereof, the settling vessel configured to receive a controlled volume VF of fluid from which the sample is to be extracted;
the air bleed port (160) of the inner tube portion coupled to an air bleed valve (161), operable between a first initial closed state when no air is released and a second open state when air is released;
the sample extraction device configured such that in use in a filling and settling mode, when the settling vessel is filled, the outer tube portion (155) in fluid communication with the settling vessel is filled with fluid to the level of the overflow outlet (151), and air is captured in the inner tube portion (153) between the air bleed port (160) and a surface of the fluid proximate the lower end of the inner tube portion (153); and
wherein in a sample extraction mode, a release of air from the inner tube portion, upon operation of the air bleed valve (161), results in a displacement of settled fluid into the inner tube and to the sample inlet (156) of the analyser (200), sample S being extracted via the sample inlet from the fluid in the inner tube.

The sample containment tube is configured such that upon release of air from the inner tube portion a resulting pressure differential between the fluid in the inner and outer tube portions causes the inner tube portion to at least partially fill such that the levels equalise. After extraction of the sample from the inner tube portion, the fluid levels in the outer and inner tube portions will again equalise.

According to an embodiment of the first aspect, the outer tube is arranged concentrically about and extending in a direction generally parallel to the inner tube, wherein the inner tube defines a first fluid containment chamber and the outer tube defines a second fluid containment chamber.

According to an embodiment of the first aspect, the sample extraction device defining a vessel head comprising first and second fluid containment chambers defined by said inner tube portion and outer tube portion respectively.

According to an embodiment of the first aspect, the vessel head comprises a double wall vessel, the wall of the inner tube portion defines a dividing wall located between and separating the inner first and second outer fluid containment chambers. The first and second fluid containment chambers may be arranged concentrically about a central axis of the sample extraction device. In use the device is arranged substantially vertically, the central axis defining vertical Z axis of the device. Further, as defined in the claims the sample extraction device is coupled to the setting vessel at the lower ends of the tube portions/fluid containment chambers and as such in use is arranged in an essentially inverted configuration relative to the fluid body in the settling vessel.

According to an embodiment of the first aspect, the sample extraction device is formed for coupling to a settling vessel or integrally formed with a settling vessel. The coupling may be for example by a thread coupling, friction fit coupling. Corresponding coupling features may be provided on the settling vessel and device.

According to an embodiment of the first aspect, the settled fluid provided to the inner tube comprises a supernatant.

According to an embodiment of the first aspect, the sample extraction device is configured for coupling in-line with an analyser wherein the sample is provided to the analyser for online and/or in situ analysis.

According to an embodiment of the first aspect, the device is configured such that the sample inlet (156) of the analyser, located within the inner tube portion, does not contact fluid from the settling vessel during the filling and settling mode of operation.

According to an embodiment of the first aspect, the sample containment tube is configured such that the sample inlet located in the inner tube portion and accordingly such that the sample inlet (156) is protected from exposure to the fluid sample during a filling stage of the settling vessel (130). The inner tube portion defines a containment tube for the sample filter 156. The sample inlet may further comprise a sample inlet filter. The filter advantageously prevent the ingress of contaminants or solids that may clog the sample inlet.

According to an embodiment of the first aspect, the sample extraction device (150) is configured to fill, at the lower end (158-1) with fluid from the upper end of the setting vessel. The fluid comprises a supernatant.

The arrangement advantageously works to prevent scum formed near a surface of the supernatant within the sample containment tube from entering the filter containment tube.

According to an embodiment of the first aspect, the overflow outlet is configured to remove excess fluid received from the settling vessel. The arrangement allows regulation of the volume provided to the settling vessel and sample extraction device. The overflow outlet is located adjacent or proximate the upper end 159-2 of the sample containment tube 150.

According to an embodiment of the first aspect, the sample device (150) according to any preceding claim, wherein the air bleed valve is operable to effect a release of air after a predetermined, settling period of time, where the settling period of time is in the range of 3 to 30 minutes, preferably 4 minutes.

According to an embodiment of the first aspect, the settling period of time is the period of time required for the volume of the mixed liquor received in the settling vessel to settle. This period of time may be pre-determined for one or more fluid sample types, and may be predetermined based on calibration, or based on other visual or alternative sensing that the separation has occurred.

According to a further aspect there is provided sample extraction device (150) configured for use in extracting a sample S for provision to an analyser (200) for analysis, the device comprising:
an inner tube portion (153) having an upper end (159-1) and a lower end (158-1);
an outer tube portion (155) having an upper end (159-2) and a lower end (158-2);
an overflow (151) outlet;
   wherein the lower ends (158) of each the outer tube portion and the inner tube portion are configured for coupling to a settling vessel (130) such that the sample containment tube (150) is in fluid communication with the settling vessel (130), the settling vessel configured to receive a controlled volume *VF* of fluid from which the sample is to be extracted; and
wherein in use in a first initial settling mode the inner tube portion (153) is at least partially filled with air such that a sample inlet (156) of an analyser, located within the inner tube portion, does not contact fluid from the settling vessel, and wherein the outer tube portion (155) is at least partially filled with fluid received from the settling vessel (130) to the overflow outlet (151) level; and
wherein in a second sampling mode an air bleed valve coupled to an air bleed port of the inner tube portion is opened to allow the release of air from the inner tube portion creating a pressure differential between fluid in the outer tube and the inner tube which in response is filled with fluid to at least the level of the inlet filter (156); and
wherein a sample of the fluid from the inner tube portion is extracted via the inlet filter (156) of the analyser and provided to the analyser for analysis.

The embodiments of the first aspect may be combined with the arrangement of the second aspect.

According to a third aspect there is provided pre-settling, sample extraction, and sample analysis system (100) for extracting a sample from a mixed liquor contained in an aeration tank (400) for automated sample analysis, the system comprising:
a settling vessel (130) configured to receive a fluid sample from the aeration tank, wherein the fluid sample in settled in the settling vessel for a predetermined settling time period;
a sample extraction device (150) according to any of claims 1 to 8, the sample extraction device configured to receive a portion of the fluid sample contained in the settling vessel (130);
an analyser (200) coupled to the system (100);
a control unit (170) configured to:
   activate a main pump (120) to supply the sample from the aeration tank to the settling vessel (120) and the sample containment tube (150);
   activate the air bleed valve (161) coupled to the inner tube portion (153) to trap air within the inner tube portion (153) while the outer tube portion (155) of the sample containment tube (150) fills with sample received from the settling vessel;
   de-activate, after a settling period of time, the air bleed valve (161) to affect a release of air from the inner tube portion providing for a displacement of settled fluid to the inner tube portion (153);
   extract, via the sample inlet (156), a fluid sample from the fluid contained within the inner tube portion (153) for analysis; and
   transfer the extracted fluid sample to the analyser (200) for analysis.

In one embodiment of third aspect, the analysis comprises an online and in-situ fluid analysis. The analysis may also comprise an online or in-situ fluid analysis. The analyser is provided arrangement coupled to and in-line with the sample extraction device. The system comprises a self-contained and integrated system configured to preform an end to end process of sample extraction through to analysis. Further the system which comprises a settling vessel, sample extraction device, controller and analyser is configured to operate in an automated manner to perform analysis of sample extracted at a remote location over a sampling period of time. The time period may be an extended period of time. The system provides a robust low maintenance system that is configured to repeat sampling at a remote location over a period of time.

In one embodiment of third aspect, the volume of the settling vessel (130) is in a range of 2 - 15 litres, preferably 4-10 litres.

In one embodiment of third aspect the setting vessel (130) is formed having a neck portion (131) that is narrower, or tapered, relative to a main body portion (132).

The arrangement allows for a separation distance between the interface between the settled fluid and sludge and assist in avoiding contamination of the sample inlet.

In one embodiment of third aspect the settling vessel (130) having a ratio of a cross sectional area at an upper of the vessel to a cross sectional area at a widest portion of the vessel in a range of 1/10 to 1/25, preferably having a ratio of 1/10.

In one embodiment of third aspect the sample containment tube (150) is configured to mechanically couple to the settling vessel (130) and wherein a fluidically sealed connection is formed between the sample containment tube (150) and the settling vessel (130) when coupled.

In one embodiment of third aspect the pre-settling system (100) is a portable pre-settling system (100) configured to be installed at a remote location/or on-site at a water treatment facility. The system may be communicatively connected to a remote data store or computer system to allow real-time storage and viewing of the result data generated by the analyser.

According to a fourth aspect there is provided a method performed by a pre-treatment settler, sample extraction and analysis system (100) of the third aspect, for automated analysis of a sample extracted from a water treatment system comprising a settling vessel, a controller, a sample extraction device, the method comprising:
actuating a delivery valve (118) of the system (100), wherein the delivery valve (118) is actuated to provide a controlled volume of fluid from the aeration tank (400) to the settling vessel;
operating an air bleed valve (161) connected to the sample extraction device (150) to prevent the release of air;
filling, via the delivery valve (118), the settling vessel (130) with a controlled volume of fluid from the aeration tank (400);
allowing the sample to settle within the settling vessel (130) and sample extraction device (150) for a settling period;
transferring, a portion of the settled sample to the inner tube portion (153) by operating the air bleed valve (161) connected to the sample extraction device (150) to affect a release of air from the inner tube portion of the sample extraction device;
extracting, via the sample inlet (156) within the inner tube portion (153) of the sample containment tube (150), a portion of the sample for analysis;
transferring, via a fluid line (157) coupled between the sample inlet (156) and an analyser (200), the portion of the sample for analysis.

According to one embodiment, the fluid provided to the inner tube portion is a supernatant. According to one embodiment, the air bleed valve is deactivated after a predetermined settling time period - or when a predetermined state of settlement is reached.

According to one embodiment, a portion of the settled sample is provided to the inner tube portion from the outer tube portion upon the release of air from the inner tube portion.

### Brief description of the drawings:

Preferred embodiment of the present invention will now be described, by way of example only, with reference to the accompanying drawings, in which:
Figure 1 shows an exemplary arrangement of the pre-settling and sampling system for analyser sample preparation according to embodiments of the present invention;
Figure 2 shows an exemplary arrangement of the pre-settling and sampling system for analyser sample preparation of Figure 1 defining an alignment axis, z-axis, according to embodiments of the present invention;

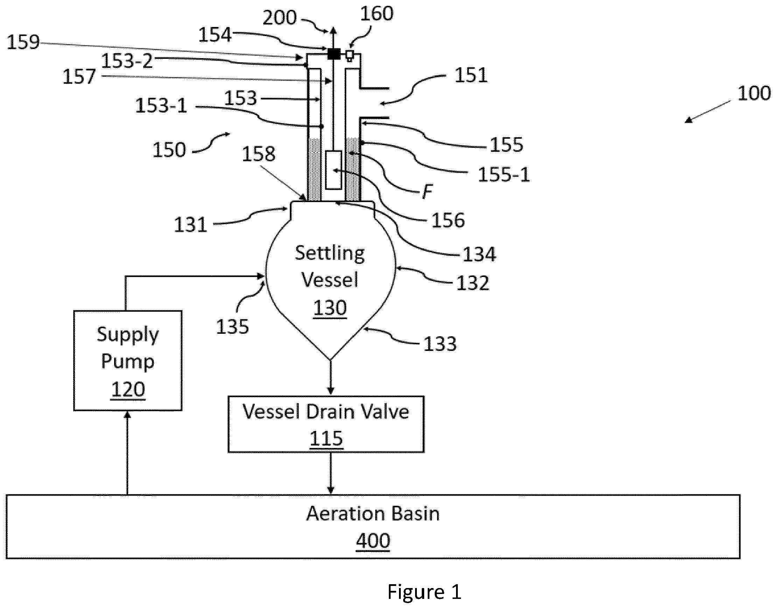

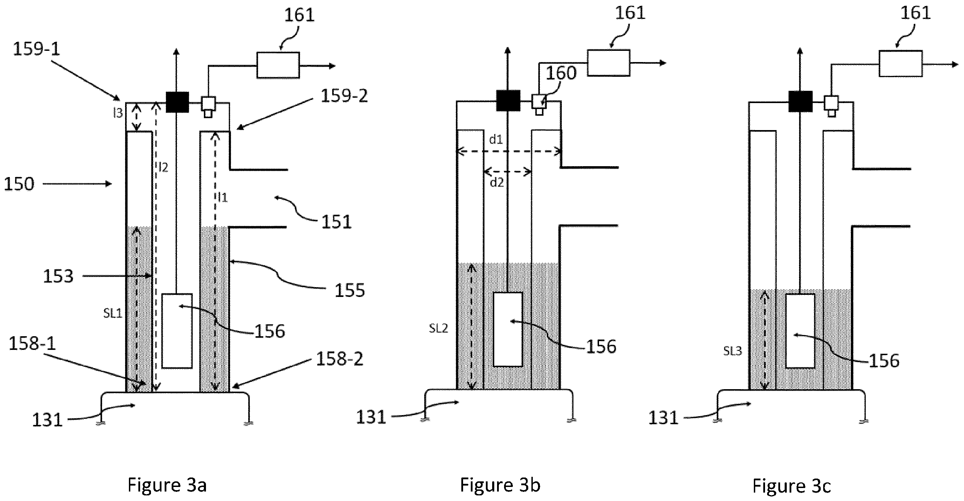

Figures 3a - 3b shows an exemplary arrangements of the sample containment tube of the pre-settling and sampling system of Figure 1 in the filling and settling mode and sample extraction mode of operation; Figure 3a, shows the arrangement in the filling and settling mode when the settling vessel is filled to the overflow outlet level and air bleed valve closed; Figures 3b and 3c show the arrangement when the settling vessel is filled and the air bleed valve is opened and air is displaced from the inner tube portion which at least partially fills with fluid and the pressure differential between the fluid in the inner and outer tube portions equalizes;
Figure 4 shows the pre-settling and sampling system for analyser sample preparation of Figure 1 located within a housing for deployment on site at a sampling location according to embodiments of the present invention;
Figure 5 shows an exemplary arrangement of the fluid connections and the electrical/control connection lines between the various components of the pre-settling system for analyser sample preparation according to embodiments of the present invention;
Figure 6 shows an exemplary arrangement of a timing diagram of the control signals for components of the pre-settling and sampling system for analyser sample preparation according to embodiments of the present invention; and
Figure 7 shows an exemplified process flow for sample extraction using the pre-settling and sampling system for analyser sample preparation according to embodiments of the present invention.

### Detailed Description

The present invention will be illustrated using the exemplified embodiments shown in figures 1 to 7 which will be described in more detail below. It should be noted that any references made to dimensions are only indicative and do not restrict the invention in any way. While this invention has been shown and described with reference to certain illustrated embodiments thereof, it will be understood by those skilled in the art that various changes in form and details may be made therein without departing from the scope of the invention encompassed by the appended claims. Furthermore, while the invention has been described with reference to particular exemplary arrangements, it will be appreciated by those skilled in the art that changes in the form and details may be made to facilitate other types of performance monitoring system without departing from the scope of the invention encompassed by the appended claims.

Embodiments of the present invention take advantage of the inherent settleability of the activated sludge mixed liquor suspended solids, MLSS, of the aeration tank, also referred to in the specification as the mixed liquor. The settleability relates to the settling characteristics of the mixed liquor and depends on the quality or state of the mixed liquor. The Activated Sludge Process, ASP, includes the use of aeration and the addition of chemical agents such as coagulants and flocculants (e.g. ferric sulphate) to the sludge plant influent to assist in accelerating the settling of suspended solid matter within the matrix. When a sample of this MLSS is extracted from the ASP process and left undisturbed i.e. allowed to settle for a period of time, the suspended solids content can be observed to settle to the bottom of the sample container under gravity to form a sludge, while a relatively clear supernatant layer can be observed to form at the top of the container. The supernatant volume increases with time as settling progresses over a period of time. A similar process may be used as part of the plant treatment process whereby a secondary clarifier is used to settle out the sludge from the aeration basin output.

Figure 1 shows an example of a mixed liquid extraction system 100 according to embodiments of the present invention. The system 100 may be a portable system configured to be installed and operated at sampling site or sampling location for example a waste water treatment plant. The system 100 is further fully self-contained and further comprises control means for operation of the system as required.

Exemplary arrangements of embodiments of the specification are configured to provide a means for extracting a sample from an aeration tank 400 of a treatment plant for automated online and in-situ fluid sample analysis. The sample in particular comprises a fluid sample for example a supernatant that is obtained after the mixed liquor settles for a period of time.

In an embodiment, the present invention relates to a system and a method of extracting, by a containment filter fluidly coupled to a settling vessel, a supernatant from a mixed liquor sample for automated online and in-situ water sample analysis.

The system 100 is further configurable to repeatedly extract a fluid sample at user-configurable time frames or automated time frames, as required. The arrangements of the embodiments of the specification are focused on obtaining and extracting the required sample and transferring that sample to an analyser 200 for analysis. The analyser 200 may be used to determine whether analytes in fluid samples from the aeration tank, or basin, 400 are within prescribed limits. This determination process is not the focus of the exemplary arrangements which are directed to providing a controlled extraction of the sample, which is a fluid sample, and which in the exemplary arrangements comprises a supernatant, and providing the sample to an analyser. It will be appreciated that while the arrangements of the specification and system 100 are described herein in particular as configured for use for testing samples from an aeration tank, it could also be deployed in other applications and that the fluid sampling could be used to deliver samples to different analyser types.

Referring to Figure 1 initially, the pre-settling and sampling system 100 comprises a supply pump, or main pump, 120 and a settling vessel 130. The settling vessel 130 is configured for use in separating the mixed liquor, in particular for separating the supernatant from the sludge of the mixed liquor. The exemplary arrangements of the specification further provide a sample containment tube 150 locatable between the settling vessel 130 and analyser 200. The sample containment tube 150 is also referred to in the present specification as the sample extraction device 150 or vessel head. The sample containment tube 150 is configured to provide a controlled extraction of a sample for online and/or *in-situ* sample analysis at an analyser 200. In the exemplary arrangements illustrated, the sample comprises the supernatant that separates from the settled mixed liquor, and the sample is provided for online and *in situ* water or supernatant sample analysis at the analyser 200. A control system 170 is configured to control the sampling, extraction and analysis and in particular to control and synchronise operations of the system components including the pump 120, valves 115, 118, air bleed valve 160/161, and analyser 200, is also provided.

In the exemplary arrangement of the drawings, sample containment tube 150 comprises an outer tube portion 155 and an inner tube portion 153. The outer and inner tubes are concentric and arranged about a common central axis, in the drawings shown in the vertical Z direction. The central axis of the sample containment tube, Z axis as shown, also defines the longitudinal axis of the sample containment tube 150. Each of the inner and outer tubes extend longitudinally from the respective upper ends to the respective lower ends thereof.

The outer tube portion has a diameter d1 and length 11. The inner tube portion has a first diameter d2 less than that of the outer tube portion. The inner tube portion further comprises a step form 153-2 at the upper end thereof. The step 153-2 defines a wider upper portion of the inner tube having a second diameter d1, generally equal to that of the outer tube portion. The outer tube portion is arranged circumferentially about the inner tube portion between the step 153-2 and the lower end 158-1. The step 153-2 defines a receiver or seat for the outer tube portion. The inner and outer tube portions may be formed as a unit or unitary structure. Alternatively the two tube portions may be provided separately for coupling together. The inner tube portion has a length or vertical extent I2 greater that the length or vertical extent I1 of the outer tube portion.

In the exemplary arrangement shown, the inner and outer tube portions each have a generally cylindrical form and the outer longitudinal wall 153-1 of the inner tube portion defines the inner wall of the outer tube portion 155. The sample containment tube 150 accordingly also has a generally cylindrical form. It will be appreciated that containment tubes of other suitable forms cross-sectional shape or geometries may also be provided. Key features include the arrangement of the inner and outer tubes configured such that a pressure differential can be created therebetween in operation.

The sample containment tube 150 is also referred to as sample extraction device 150 and a vessel head in the specification. In the arrangement of the system 100, the sample containment tube is configured for coupling to the settling vessel 130 at an upper end 131, 134 thereof defining a vessel head of the fluid settling vessel. In the arrangement of the drawings the sample containment tube 150 is arranged in a generally vertical orientation in use and coupled to the settling vessel at a vertical axis thereof and at a level generally above the settling vessel.

Outer wall 155-1 of the outer tube portion 155 defines a portion of the outer wall of the sample containment tube 150. The inner tube portion 153 comprises an upper end 159-1 and lower end 158-1. The outer tube portion 155 comprises an upper end 159-2 and a lower end 158-2. The upper ends 159-1, 159-2 are closed, and the lower ends 158-1 and 158-2 are open such that each of the inner tube potion and the outer tube portion defines an interior volume for receiving a fluid via the lower openings thereof. The outer tube portion 155 further comprises an overflow outlet 151 located between the upper 159-2 and lower 158-2 ends thereof. In the arrangement of the drawings the overflow outlet 151 is located near the upper end of the outer tube portion 155.

The sample containment tube 150 is connectable at the lower end 158 thereof, and in particular at the lower end 158-2 of the outer tube portion to the settling vessel at an upper opening 134 of the settling vessel 130.

The lower ends 158-1, and 158-2 may be coincident or extend to the same vertical position or level when the sample containment tube150 is coupled to a settling vessel 130. When the sample containment tube is coupled to the settling vessel - the lower end 158-1 of the inner tube portion is located proximate an upper surface of the fluid in the settling vessel, when filled.

The outer tube is arranged concentrically with and extending in a direction generally parallel to the inner tube, wherein the inner tube defines a first fluid containment chamber and the outer tube defines a second fluid containment chamber. The sample extraction device defining a vessel head comprising first and second fluid containment chambers defined by said inner tube portion and outer tube portion respectively.

Air bleed port 160, also referred to in the specification as air outlet, and air bleed valve 161 are provided at the upper end 159-1 of the inner tube portion. The inner tube portion 153 is configured to receive a sample inlet filter 156 for extracting a fluid sample for the analyser during the sample extraction phase of operation. In effect the sample inlet filter is locatable within the inner tube portion spaced apart from and above the lower end of the tube.

The sample containment tube 150 is configured to be located between the setting vessel 130 and the analyser 200 in use. This arrangement advantageously provides protection for the analyser and for the controlled extraction of samples and provision of samples to the analyser.

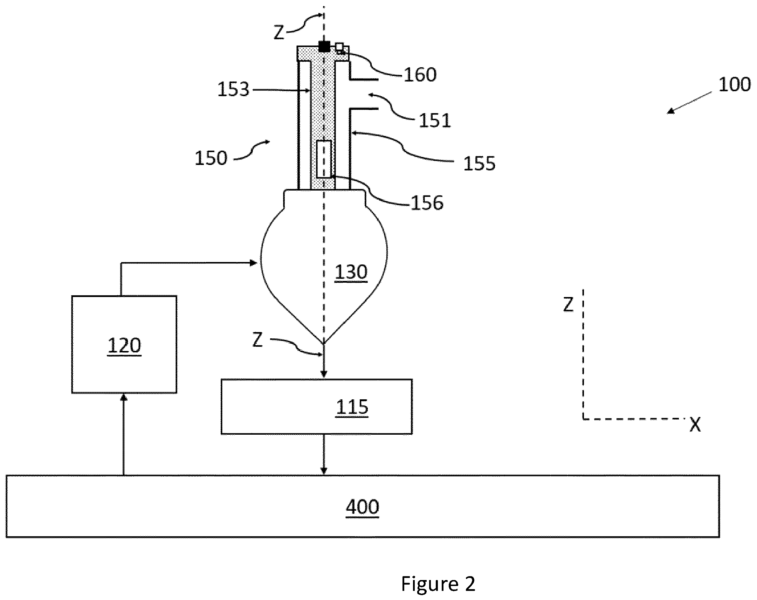

In one arrangement as shown in Figure 2, the sample containment tube 150 is configured for coupling to the settling vessel 130. In some arrangements the sample containment tube 150 may be formed as a unitary piece together with the settling vessel 130.

The system is configured such that in use, the sample containment tube 150 extends generally upwardly, from the neck 131 of the settling vessel 130, relative to the settling vessel 130. The arrangement shows the sample containment tube 150 arranged aligned with the central axis, Z, of the settling vessel 130. It will be appreciated that the the sample containment tube 150 may be configured so that it is offset from the central longitudinal axis, Z, of the settling vessel 130 and/or extending at an angle relative to the vertical Z axis, of the settling vessel 130.

The sample containment tube 150 is configured to be coupled to settling vessel 130 and in fluid communication with the settling vessel at the lower ends of the inner and outer tube portions thereof. The settling vessel is configured to receive a controlled volume VF of fluid from which the sample is to be obtained. The settling vessel 130 is filled at an inlet 135 located at a level below the upper portion 131, 134 at which the sample containment tube is located.

The air bleed valve 161 of the inner tube portion is controllably operable between a first closed position and a second open position. In a first filling and settling mode of operation of the system 100 the valve is deactivated such that the air bleed port is closed and no air escapes or passes out through the air bleed port of the inner tube portion. In a second sample extraction mode of operation, the valve is activated such that air can escape or flow out from the inner tube portion via the port 160.

In the filling and settling mode, the settling vessel 130 is filled to a fill level, and the outer tube portion 155 in fluid communication with the settling vessel is also filled with fluid to the level of the overflow outlet 151. At the same time, with the air bleed valve 161 deactivated and port 160 closed, air is captured in the inner tube portion 153 between the air bleed port 160 and a surface of the body of fluid in the settling vessel 130 proximate the lower end 158-1 of the inner tube portion 153. At this time, the sample inlet 156 located in the inner tube portion 153 does not contact the fluid, but rather is surrounded by air. It will be appreciated that the arrangement can also be described as providing capture of a volume of air between the surface of the fluid in the settling vessel and proximate the lower ends of the sample inner tube portion and the air outlet valve or air outlet.

In the sample extraction mode, after a predetermined fluid settling period of time, the air bleed valve 161 is activated to allow the release air from the inner tube portion 153 at port 160 resulting in a displacement of settled fluid into the inner tube portion, which fills to at least the level of the sample inlet 156 of the analyser 200.

This displacement results from the creation, by the release of air, of a pressure differential between the fluid in the outer 155 and inner tube 153 portions. The fluid levels SL2 in both tube portions equalise - see figure 3b before the sample is extracted, and equalise again to a lower level SL3 as shown in figure 3c, after extraction of the sample. As noted from the illustrations the level in each of the inner and outer tube equalises to SL3 in Figure 3c.

In a first stage of operation, the filling and settling, the mixed liquor sample is received in the outer tube portion only. The inner tube portion is at least partially filled with air such that the mixed liquor sample does not fill into the inner tube portion and in particular does not contact the sample filter 156. In a preferred arrangement the inner tube portion is essentially or substantially filled with air. Thus, during the initial settling stage, the inner tube portion 153 is at least partially filled with air such that the inlet filter 156 of the analyser, located within the inner tube portion, does not contact fluid from the settling vessel. At the same time, the outer tube portion 155, open to atmospheric pressure at the overflow outlet 151 does not trap air and is at least partially filled with fluid received from the settling vessel 130 to the overflow outlet 151 level.

In the active sampling stage the air bleed valve 161 is activated and the air bleed port 160, also referred to as the outlet, is opened, to allow the release of air from the inner tube portion creating a pressure differential between fluid in the outer tube and the inner tube which in response is filled with fluid to at least the level of the inlet filter 156.

This allows for a sample to be collected for transfer to the analyser 200. The air bleed valve 161 may be provided coupled to the air outlet or bleed port 160 of the inner tube portion.

To perform an analysis of the mixed liquor, a sample S needs to be obtained from an aeration tank 400 at the sludge plant.

Pre-settling and sampling system 100, can be used to obtain such a sample for online and/or *in-situ* sample analysis. The pre-settling system 100 comprises a supply pump 120 and a settling vessel 130 arranged in fluid communication. The pre-settling system 100 is deployed at a treatment plant such that the settling vessel 130 can be fluidly coupled or in fluid communication with the aeration tank 400 at the plant.

A mixed liquor sample is provided to the settling vessel 130 using the supply pump 120 which is operable to transfer a controlled volume of the mixed liquor from the aeration tank 400 to the settling vessel 130 to which it is fluidly coupled. The volume provided to the settling vessel by the pump is predefined for the particular arrangement and configuration of the system 100. In particular, the volume will depend on the volume of the settling vessel. During use in a settling and sampling run the settling vessel is filled to the overflow. The supply pump 120 may be a self-priming pump. The pump operation is controlled by control system 170 which also controls delivery valve 118 and drain valve 115.

With reference to Figures 1, 2, 4 and 5, the settling vessel 130 of the pre-settling and sampling system 100 is configured to allow the mixed liquor that has been received therein to settle for a period of time prior to the action to extract a sample *S*, for sample analysis.

Factors that may affect the settling process include the shape and volume of the settling vessel 130. The setting vessel 130 is configured to support settling to allow extraction of the sample. In an exemplary arrangement a container or vessel having a volume in the range of 2 to 15 Litres may be provided. In an exemplary arrangement of an embodiment of the specification, a vessel having a volume of 4 Litres may be provided, which supports provision of a compact arrangement of system 100. In other exemplary arrangements a vessel of volume 10 Litres may be provided as supporting a compact system form.

Settling vessel 130 of exemplary Figure 1 is further formed having an upper, or neck, portion 131 that is narrower relative to the main body portion 132, the lower base portion 133 is also narrower than the main body portion 132. The neck portion 131 effectively has a smaller cross-sectional area or smaller diameter than the main body portion 132. In the exemplary arrangement, the neck portion has a rounded or curved form and the upper and lower portions of the vessel taper in form from the main body portion 132 to the neck 131 and base 133.

A settling vessel 130 having a ratio of the cross-sectional area at the top 131 of the vessel 130 to the cross-sectional area at the widest portion of the vessel in the range of 1/10 to 1/25 may be provided. In a most preferred arrangement, a sampling vessel having a ratio of 1/10 may be provided. The settling vessel according to the specification and system 100 is thus configured to provide for liquid in the uppermost portions of the settling vessel to clear quickly as the mixed liquor volume contained therein settles.

As noted, the settling vessel 130 may further be formed to have a rounded or tapered or curved base portion 133. The rounded or curved base portion form advantageously aids in providing effective drainage of the tank under gravity. It will be appreciated that settling vessels of different form may be provided.

As an example of the use of system 100 and a settling vessel 130 according to the system and methods of the present specification, a settling vessel 130 according to Figure 1 was provided having a total volume of 10 Litres. It was found advantageously that a volume of 0.5 litres, 0.5L, of supernatant was formed within 10 minutes from a total liquor volume of 10 Litres.

A further advantage of a settling vessel 130 having a narrow upper section is that the arrangement provides a well-defined clearance separation or clearance distance between a supernatant-sludge interface line and the sample containment tube and sample filter during the extraction of supernatant. The supernatant-sludge interface line is that line that forms between the supernatant that settles to the upper portion of the container and the sludge from which it has separated. A separation between the two different fluids may be visibly discernible in the settling tank 130. The settling vessel 130 is configured to have a relatively narrower neck portion 131 such that the separation line between supernatant and sludge is presented at a lower level relative to the neck, than in a settling vessel that does not have such a narrower portion. Based on the configuration of the vessel, the interface line is located to provide a separation or clearance between the sample inlet156 and the separation line. As such, the filter located proximate to the neck portion is positioned such that it is well clear of the sludge before a sample is drawn, via the filter, for analysis. If the separation line is not adequately spaced apart from the bottom of the filter or neck portion, there is an increased risk that sludge will be drawn onto the filter thereby potentially clogging the filter, and affecting any sample analysis and resulting in possible downtime to the system and incurring maintenance costs. The arrangement shown in Figure 1 avoids these issues.

With reference to Figures 1 to 5, the pre-settling and sampling system 100 comprises a sample containment tube 150, also referred to as a filter containment tube. The sample containment tube 150 may also be referred to as a vessel head when connected, or coupled to the settling vessel 130. The sample containment tube 150 may be formed such that it is configured to be mechanically coupled to the settling vessel 130 such that a fluidically sealed connection is formed between the sample containment tube 150 and the settling vessel 130 when coupled together. The sample containment tube 150 may be coupled to the settling vessel 130 via, for example but not limited to, a screw type arrangement where the sample containment tube 150 is screwed into a receiving section of the settling vessel 130. For example in a threaded pipe connection type arrangement. In other arrangements the sample containment tube may be friction fitted or clamp fitted onto the settling vessel. Any suitable means or form of providing a coupling arrangement of the sample containment tube 150 to the settling vessel 130 may be employed. In other arrangement, the sample containment tube 150 and the settling vessel 130 may be formed as a unitary integral structure. In the arrangement shown in Figure 1, a received fluid is shown within the outer tube portion of the sample containment tube 150.

The sample containment tube 150 of Figures 1 to 5 comprises the following features:
151 Overflow outlet
153 Inner tube portion (filter locatable therein), 153-1 longitudinal wall outer or external wall of the inner tube portion, 153-2 stepped end of inner tube portion
154 Bulkhead tubing connection
155 Outer tube portion, 155-1 longitudinal or external wall of outer tube portion
156 Sample inlet also referred to as sample inlet filter or filter in the specification
157 Sample supply/delivery line from sample inlet/sample inlet filter to analyser
158 Lower end of sample containment tube/lower ends 158-1, 158-2 of each of the inner and outer tube portions
159 Upper end of sample containment tube//upper ends 159-1, 159-2 of each of the inner and outer tube portions
160 Air bleed port or outlet of inner tube portion 153 of the sample containment tube 150, may be coupled to an air bleed valve 161 controllable by controller 170

In an exemplary arrangement of embodiments of the specification, the walls of the inner tube portion 153 comprises rigid polyvinyl chloride (PVC) pipe, such as unplasticized polyvinyl chloride(uPVC) and the outer tube portion comprises rigid polyvinyl chloride (PVC) pipe, such as unplasticized polyvinyl chloride (uPVC).

In an arrangement where the sample containment tube 150 is separate from the settling vessel 130, an outer wall, 155-1, of the outer tube portion 155 of the sample containment tube 150 may be mechanically connected to the settling vessel 130. Example means for connection have been discussed above.

In an exemplary arrangement, the volume of the inner tube portion 153 may be of the order of 100 to 120 millilitres, 100 to 120 ml. The volume of the outer tube may be of the order of 200 to 260 millilitres, 200 to 260 ml.

As an example, in one exemplary arrangement, an inner tube is provided having a volume of the order of 108 millilitres, 108 ml, and the outer tube provided having a volume of the order of 232 millilitres, 232 ml. Thus, the ratio of volume between the inner and outer portions may be in the range of about 1:2.

One aim of the use of the filter or sample containment tube 150 is to protect the inlet filter 156 coupled to the analyser 200, via fluid line 157, such that the filter 156 is not submerged in the raw mixed liquor during the vessel 130 filling and settling stages.

By virtue of the arrangement of the present specification and the operation thereof to obtain a sample, the filter 156 is exposed only to the clear supernatant and only for a relatively short duration of time, i.e. while the inner tube portion 153 of the sample containment tube 150 fills with supernatant and the sample is being extracted. After the sample is extracted, the sample containment tube is drained and the settling vessel 130 is drained. While the mixed liquor settles during the settling phase or state of operation of the system, the inner tube portion is at least partially filled /or filled with air and thus the inlet filter is not exposed to the mixed liquor. With reference to the advantages of the control of exposure of filter 156 to the sample being limited as supported by the system 100 according to the specification - it is noted that a long duration exposure of the inlet filter 156 to the raw mixed liquor would cause the filter to quickly clog/block due to the solid content. These solids have high levels of biological microbes (bacteria, protozoa) which are naturally used in the ASP as part of the nitrification process. Further in a case that the inlet filter 156 was arranged permanently submerged in the sample accelerates the growth of biofilms (e.g. algae) on the filter surfaces which in turn leads the filter blocking more quickly.

Therefore, the operation of the system 100 and the arrangement of the sample containment tube 150 and system 100 such as to provide for filling of the inner tube portion 153 of the containment tube 150 from the lower end 158 after the mixed liquor has settled and subsequent drainage of the inner tube addresses and obviates issues arising from exposure of the filter 156 to the mixed liquor. By virtue of the arrangements of the specification, the inlet filter is exposed at intervals to a sample of the supernatant after settling of the mixed liquor. Further, the arrangements of the specification advantageously prevent any scum/foam that might sit on the top of the supernatant from entering the containment tube. The sample is extracted via the filer 156 and sample supply line 157 to the analyser 200.

With reference to Figure 3, shown is a method of operation of the system 100 and the different states of the filter containment tube or sample containment tube 150. The filter containment tube 150 is configured to provide a liquid pressure differential between the inner and outer tube portions thereof.

The inner tube portion 153 has an upper end 159-1 and a lower end 158-1. The outer tube 155 has an upper end 159-2 and a lower end 158-2. The outer tube comprises an overflow 151. The lower end of the outer tube is coupled to an upper portion of the settling vessel 130 such that the same containment tube is in fluid communication with the settling vessel.

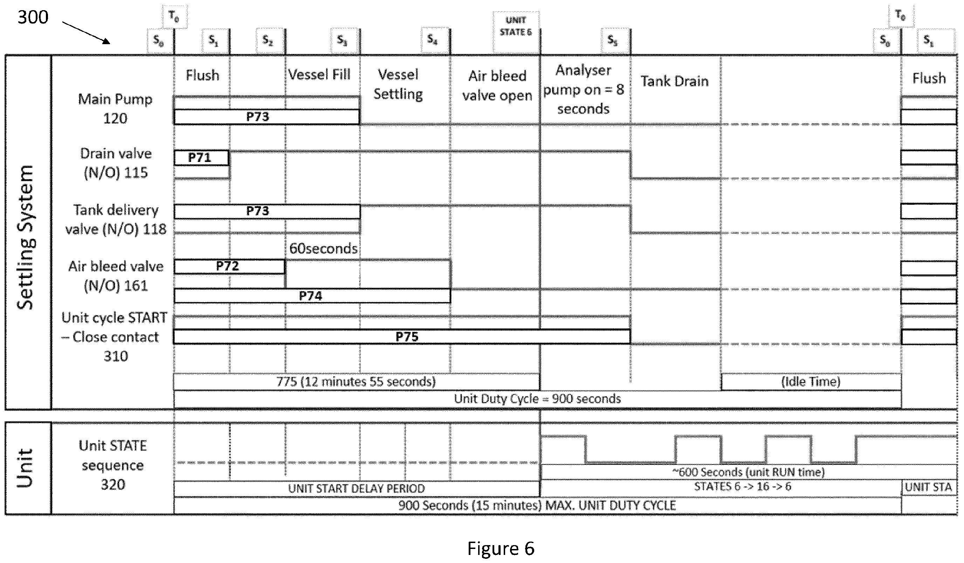

During the vessel 130 filling step, see step 420 of Figure 6, an air bleed valve 161, coupled to an air bleed port 160 located at the top 159-1 or upper end 159-1 of the inner tube portion 153 of the containment tube, is closed such that air in the inner tube portion 153 is captured therein and cannot escape, as the fluid level in the settling vessel rises, from the lower end thereof, to the level of the sample containment tube 150 and in particular to reach proximate the lower end 158-1 of the inner portion and the overflow 151 level of the outer tube portion.

In this initial vessel 130 filling step the inner tube portion is filled with air. The lower end 158-1 of the inner tube 153 is in contact with the fluid in the settling vessel and air is contained between an upper surface portion of the fluid at the lower end of the inner tube portion and the air bleed valve.

During the filling step, and when the settling vessel is filled to contain a predefined volume of the mixed liquor, the outer tube portion 155 of the sample containment tube 150, coupled to the neck portion 131 of the settling vessel 130 fills with mixed liquor as the settling vessel is filled, and fills up to the sample containment tube overflow 151. The outer tube fills to a first level SL1.

In the arrangement of the sample containment tube 150 of Figures 3a - 3c according to embodiments of the present invention, the mixed liquor cannot enter the inner tube portion 153 of the sample containment tube 150 due to the air trapped within that inner tube portion 153 as illustrated in Figure 3a. In this way, the inlet filter 156 is not exposed to unsettled mixed liquor and is only exposed to air. After a predefined settling time, and when a clear supernatant forms at the upper end or head of the settling vessel 130, and the outer tube portion 155, the air bleed valve 161 is opened. The air captured in the inner tube portion 153 during the filling step is released. Accordingly, a pressure differential is created between the inner tube portion 153 and the outer tube portion 155, the liquid level in the outer tube portion 155 drops as illustrated in Figure 3b and the liquid in the inner tube portion 153 of the sample containment tube 150 rises until the liquid levels SL2 between the inner and outer tube portions 153, 155 equalise in level. In effect, the liquid that enters the bottom end 158-1 of the inner tube portion 153 displaces a volume of air from within the inner tube portion 153 due to the liquid pressure differential created and to equalise that liquid pressure differential.

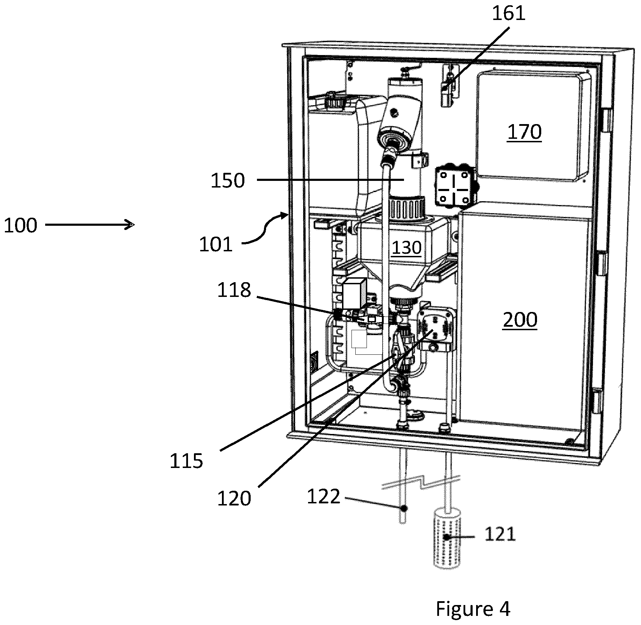

Figure 4 shows an example of a mixed liquid extraction system 100 according to embodiments of the present invention located within a housing 101. Housing 101 may be used to house the settling vessel 130, the sample containment tube 150, the control unit 170, the analyser 200, the main pump/delivery pump 120, the delivery valve 118, the drain valve 115 and the electrical and fluid lines coupling the components of the pre-settling system 100. The housing may be configured to be portable for transporting and installation at a treatment plant for example. A fluid line 121 may couple to a coarse strainer which strains the sample being obtained from an aeration tank 400 for transferring via the delivery pump 120 to the settling vessel 130. The coarse strainer is provided to ensure that coarse material is prevented from entering the settling vessel 130 when the sample is being obtained from the aeration tank/basin 400. Also shown is a fluid return line 122 for return fluid from the system 100 back to the aeration tank 400.

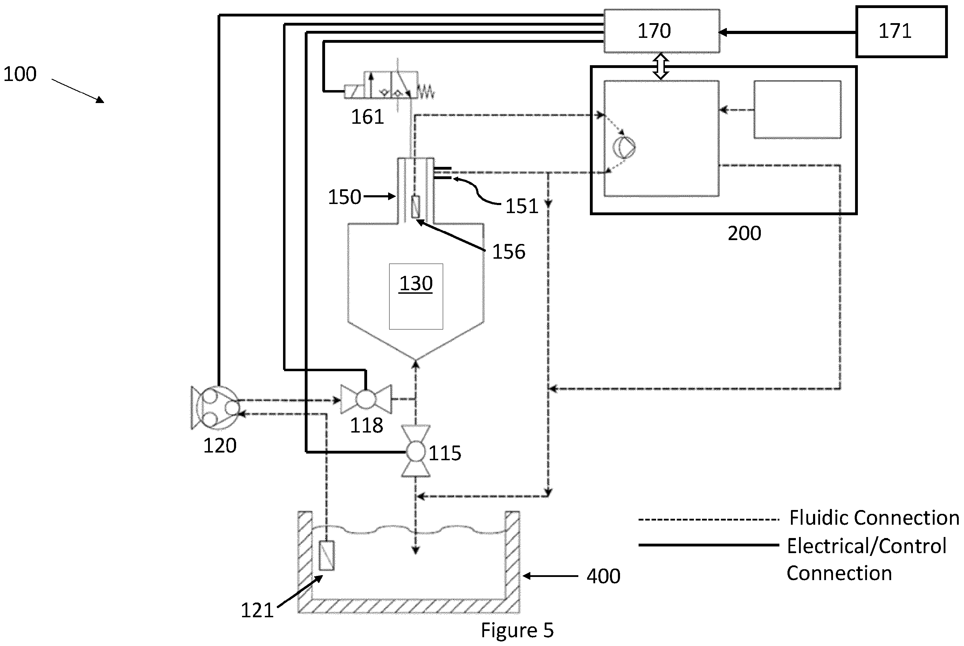

Figure 5 shows an example of a mixed liquid settling and extraction system 100 according to embodiments of the present invention with the fluidic connection lines and the electrical/control connection lines coupled to the various components of the system. In the arrangement shown, the control unit 170 receives mains power from an electrical point 171. The control unit 170 is configured to provide electrical/control connections to the analyser 200, the air bleed valve 161, the main pump/delivery pump 120, the delivery valve 118 and the drain valve 115 so as to enable the system 100 to operate to obtain a sample from the aeration tank 400 for analysis by the analyser 200 as previously described. The fluidic connections as shown provide a fluidic path from the aeration tank 400 via a fluid line coupled between the coarse strainer 121 to the main pump/delivery pump 120 which then provides this fluid sample to the settling vessel via the delivery valve 118. The fluid sample for analysis by the analyser 200 is obtained via the fluid line coupled between the filter 156 of the sample containment tube 150 and the analyser.

A fluid return path 122 to the aeration tank may be also provided via the overflow 151 of the sample containment tube 150. A fluid return path to the aeration tank is provided from the analyser 200 so as to return any analysed fluid sample back to the aeration tank 400. The fluid connections lines are configured so as to enable the fluid lines to be flushed during, for example, maintenance of the pre-settling system or post analysis of a fluid sample of aeration tank and before any subsequent sample analysis operations.

Figure 6 shows an exemplary timing diagram related to the control of operation of a number of components of the system 100 which are operable when providing a fluid volume to the settling vessel for settling and then for extracting a supernatant sample for online and/or *in-situ* sample analysis at analyser 200. The diagram is arranged showing various states and time intervals in which various components may be activated and/or deactivated to a particular state, at a given time interval, in order to enable the system to obtain a fluid sample from an aeration tank, transfer that sample to the settling vessel and the filter containment tube and then extract a supernatant for online and *in-situ* water sample analysis at analyser 200.

T₀ defines the status of the system at time zero. The components of the settling system configured for activation and/or deactivation as shown in the timing diagram are the main pump (or the supply pump), the drain valve, the delivery valve, the air bleed valve and a unit cycle start.

The timing diagram also illustrates a number of states, S, and parameters, P, which are referenced relative to the start which is To in the illustrated arrangement. For each of the components the main pump/delivery pump/supply pump 120, the drain valve 115, the delivery valve 118, the air bleed valve 117 and a unit cycle start there is an associated configurable parameter. The main pump has a configurable parameter listed as P73, the drain valve has a configurable parameter listed as P71, the delivery valve has a configurable parameter listed as P73, the air bleed valve has a configurable parameter listed as P72 and P74, and a unit cycle start has a configurable parameter listed as P75.

In the exemplary arrangement shown, there are two timing diagrams shown. The settling system timing diagram is the timing diagram for the pre-settling system 100 while the unit timing diagram is the timing diagram for the analyser 200.

The unit state sequence of the analyser comprises at least 16 states of operation. Unit state 0 to state 6 may be defined as a unit start delay time, or unit start delay period, in which the unit is delayed from running prior to the sample main pump being turned on. This unit start delay period may be within a range of 280 to 320 seconds, preferably 300 seconds. Unit states 6 to 16 may be defined as a unit run time, or unit run period, in which the unit is running upon activation of the sample main pump, that is, the sample pump being turned on. This unit run time may be within a range of 580 to 620 seconds, preferably 600 seconds. The overall unit duty cycle which comprises the unit run time and the unit start delay time may be within 880 to 920 seconds, preferably 900 seconds.

In the arrangement shown, T0 to T0 defines a unit duty cycle of about 900 seconds. Transitions between the settling system states and their associated effect on the system are as follows: At state S0, corresponding to T0, the main pump 120 is switched ON, the drain valve 115 is OFF, the delivery valve 118 is OFF and the air bleed valve 161 is OFF. This results in a flush, or pipework flush, of the system 100 so as to clean, or flush, the fluid lines of the system.
- At state S1, the drain valve is turned ON. This actuation of the drain valve at S1 is associated with parameter P71. This results in a closure of the drain valve and therefore ends a flush of the fluidic lines.
- At state S2, the air bleed valve is turned ON. The air bleed valve is turned on for a duration of 60 seconds. This actuation of the air bleed valve at S2 is associated with parameter P72. This results in trapping of the air within the inner tube portion 153 of the sample containment tube 150 while also enabling the settling vessel 130 and the outer tube portion 155 of the sample containment tube 150 to begin filling with the mixed liquor sample being obtained from the aeration tank 400 which is being pumped via the main pump 120.
- At state S3, the main pump is turned OFF, the delivery valve is turned ON. The delivery valve being turned on prevents mixed liquor sample flowing back to the main pump. The mixed liquor sample in settling tank is then allowed to settle for a period of time. This actuation of the main pump at S3 is associated with parameter P73 and the actuation of the delivery valve at S3 is associated with parameter P73.
- At state S4, the air bleed valve is turned OFF. The air bleed valve is turned OFF such that the air bleed valve is opened. This actuation of the air bleed valve at S4 is associated with parameter P74. This enables movement of supernatant from the outer tube portion 155 to the inner tube portion 153 of the sample containment tube.
- Between states S4 and S5 of the settling system, the unit start delay period ends and a unit run time is activated. This is referred to as unit state 6 and results in a sample pump of the analyser being activated such that supernatant is extracted, via the filter 156 of the sample containment tube 150, for online and/or *in-situ* sample analysis at analyser 200. The sample pump is turned on for a period of 8 seconds to enable extraction of the supernatant from the sample containment tube 150.
- At state S5, the drain valve and the delivery valve are turned OFF and the unit cycle start 310 is actuated. The actuation of the drain valve at S5 is associated with parameter P71, the actuation of the delivery valve at S5 is associated with parameter P73 and the actuation of the unity cycle start 310 at S5 is associated with parameter P75. This enables the settling vessel 130 and the sample containment tube 150 to drain and the system enters an idle time before commencing with a subsequent unit duty cycle, that is, a subsequent extraction of a supernatant for online and/or *in-situ* sample analysis at analyser 200.

The following parameter times, relative to the start T0, may be associated with the above parameters. P71 actuated for 10 seconds, P72 actuated for 95 seconds, P73 actuated for 125 seconds, P74 actuated for 360 seconds and P75 actuated for 390 seconds.

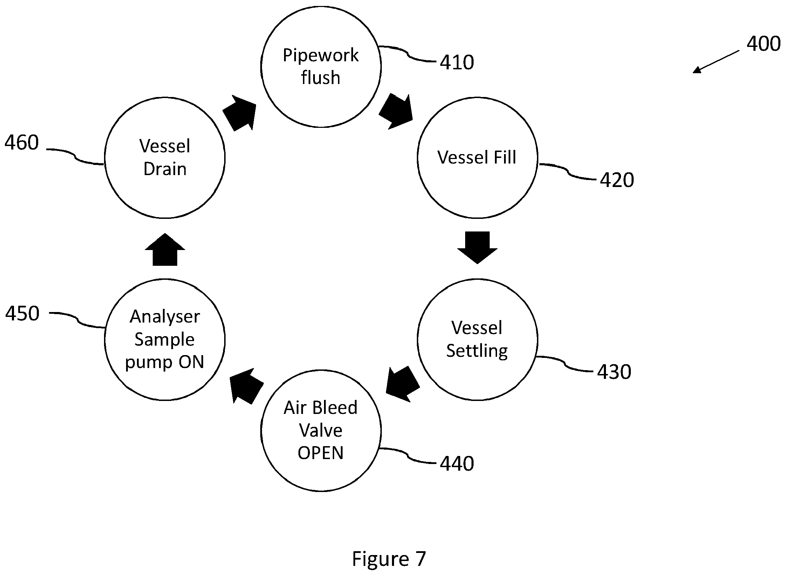

Figure 7 shows an exemplified process flow for operating stages of the pre-settling system 100. Although the process flow is shown in the form of a repeated linear flow, it will be appreciated that certain elements may be executed in parallel, or in combination with other steps, or in different order. Each stage is performed based on information obtained directly from a sensor, a component output, or derived from other information. In the pre-settling system, the control unit 170, such as but not limited to a programmable logic control (PLC), controls the actuation of the valves and pumps of the system and is configured to set up the timing sequence for the process to enable extraction of a supernatant. The control unit may also be configured to enable the system to clean the pipework of the system by flushing the system. The control unit 170 may be interfaced with an analyser 200 for online and/or in-situ sample analysis such that the analyser 200 can initiate and synchronise its sampling duty cycle with the pre-settling system 100. The control unit may be configured to control the following elements of the system:
- Drain valve 115 - actuated to allow the settling vessel 130 to fill and drain. The valve is actuated to OPEN/CLOSE positions.
- Tank delivery valve 118 - actuated to prevent any backflow on the pump-to-vessel supply line. The valve is actuated to OPEN/CLOSE positions.
- Air bleed valve 161 - actuated to trap air within the inner tube portion 153 of the filter containment tube 150, then opened to allow the air to be forced out of the inner tube portion 153 by the liquid entering at the bottom end 158. The valve is actuated to OPEN/CLOSE positions.
- Main pump/supply pump/delivery pump 120 - powered on/off to fill the sampling vessel 130 with sample from the aeration basin/tank 400.

The control of the valves and pumps may be configured such that the timing periods for each process step can be easily optimised, depending on process variables such as but not limited to sample settleability, sampling vessel 130 filling time, outer tube portion 155 filling time. The time periods may be selected or adjusted based on calibration runs.

The process flow 400 for batch sampling as illustrated in Figure 7 begins with configuring the system so as to flush 410, or clean, the pipework of the system 100. This may involve inputting a fluid into the system, the fluid may be a water solution, to enable the fluid connection lines within the system to be flushed with the fluid sample thereby cleaning the fluid connection lines prior to performing extraction of a sample for analysis by the analyser 200. The process may provide a filling 420 stage where the sampling vessel 130 is filled with a sample from an aeration tank 400 followed by a settling 430 stage where the sample obtained from the aeration tank is allowed to settle, for a settling period, in the sampling vessel 130 and, as described above, within the sample containment tube 150 thereby allowing a clear supernatant to form during the settling period. The process then moves onto the next stage where the air bleed valve 161, coupled to an air bleed port 160 of the sample containment tube 150, is actuated to an opened position and a portion of the settled sample, or supernatant, is received 440 within the inner tube portion 153 of the sample containment tube 150. To extract the supernatant within the sample containment tube 150, an extraction 450 stage enables the supernatant to be extracted from the inner tube portion 153 of the sample containment tube 150 via the filter 156 within the inner tube portion 153 as previously described. Once a supernatant has been extracted and sent to the analyser 200 for analysis, the process is followed by a draining 460 stage in which the settling vessel 130 and the sample containment tube 150 are drained such that any fluid contained therein is coupled back into the aeration tank 400 via a fluid line coupled between the sampling vessel 130, a drain valve 115 and the aeration tank 400.

An advantage of arrangements of the specification in comparison with previous system includes that convention membrane based systems are prone to clogging up and therefore requirements maintenance and continual servicing time to address such issues whereas the present invention does not clog up and therefore the system has a longer deployment time and less servicing time.

## Claims

1. A sample extraction device (150) configured for use in extracting a sample S from a fluid, for provision to an analyser (200) for analysis, the sample extraction device:
a first inner tube (153) having an upper end (159-1) and a lower end (158-1), the lower end defining an opening and the upper end defining a closed end of the inner tube (153);
a second outer tube (155) having an upper end (159-2) and a lower end (158-2), the lower end defining an opening, the outer tube comprising an overflow outlet (151);
wherein the outer tube portion (155) is arranged generally parallel to the inner tube portion (153), and wherein the lower end (158-2) of the outer tube (155) is in fluid communication with the lower end (158-1) of the inner tube (153);
an air bleed port (160) located at the upper end (159-1) of the inner tube portion (153);
a sample inlet (156) for extracting a sample for transfer to the analyser located in the inner tube between the upper and lower ends thereof;
the sample extraction device (150) configured to be coupled to a settling vessel (130) such that in use it is in fluid communication with the settling vessel at the lower ends of the inner and outer tube portions thereof, the settling vessel configured to receive a controlled volume VF of fluid from which the sample is to be extracted;
the air bleed port (160) of the inner tube portion coupled to an air bleed valve (161), operable between a first initial closed state when no air is released and a second open state when air is released;
the sample extraction device configured such that in use in a filling and settling mode, when the settling vessel is filled, the outer tube portion (155) in fluid communication with the settling vessel is filled with fluid to the level of the overflow outlet (151), and air is captured in the inner tube portion (153) between the air bleed port (160) and a surface of the fluid proximate the lower end of the inner tube portion (153); and
wherein in a sample extraction mode, a release of air from the inner tube portion, upon operation of the air bleed valve (161), results in a displacement of settled fluid into the inner tube and to the sample inlet (156) of the analyser (200), sample S being extracted via the sample inlet from the fluid in the inner tube.

2. The device of claim 1 wherein the outer tube is arranged concentrically with and extending in a direction generally parallel to the inner tube, wherein the inner tube defines a first fluid containment chamber and the outer tube defines a second fluid containment chamber.

3. The device of claim 1 or 2, the sample extraction device defining a vessel head comprising first and second fluid containment chambers defined by said inner tube portion and outer tube portion respectively.

4. The device of any preceding claim, formed for coupling to a settling vessel or integrally formed with a settling vessel.

5. The device (150) of any preceding claim, wherein the settled fluid provided to the inner tube for sample extraction comprises a supernatant.

6. The device (150) of any preceding claim, configured for coupling in-line with an analyser wherein the sample is provided to the analyser for online and/or in situ analysis.

7. The device of any preceding claim, wherein the device is configured such that the sample inlet (156) of the analyser, located within the inner tube portion, does not contact fluid from the settling vessel during the filling and settling mode of operation.

8. The device according to any preceding claim, wherein the air bleed valve is operable to affect a release of air after a predetermined settling period of time, where the settling period of time is in the range of 3 to 30 minutes, preferably 4 minutes.

9. A pre-settling, sample extraction, and sample analysis system (100) for extracting a sample from a mixed liquor contained in an aeration tank (400) for automated sample analysis, the system comprising:
a settling vessel (130) configured to receive a fluid sample from the aeration tank, wherein the fluid sample in settled in the settling vessel for a predetermined settling time period;
a sample extraction device (150) according to any of claims 1 to 8, the sample extraction device configured to receive a portion of the fluid sample contained in the settling vessel (130);
an analyser (200) coupled to the system (100);
a control unit (170) configured to:
activate a main pump (120) to supply the sample from the aeration tank to the settling vessel (120) and the sample containment tube (150);
activate the air bleed valve (161) coupled to the inner tube portion (153) to trap air within the inner tube portion (153) while the outer tube portion (155) of the sample containment tube (150) fills with sample received from the settling vessel;
de-activate, after a settling period of time, the air bleed valve (161) to affect a release of air from the inner tube portion providing for a displacement of settled fluid to the inner tube portion (153);
extract, via the sample inlet (156), a fluid sample from the fluid contained within the inner tube portion (153) for analysis; and
transfer the extracted fluid sample to the analyser (200) for analysis.

10. The system (100) according to claim 9, wherein the volume of the settling vessel (130) is in a range of 2 - 15 litres, preferably 4-10 litres.

11. The system (100) according to claims 9 or 10, wherein the setting vessel (130) is formed having a neck portion (131) that is narrower, or tapered, relative to a main body portion (132).

12. The system (100) according to claims 9 to 11, the settling vessel (130) having a ratio of a cross sectional area at an upper portion (131) of the vessel to a cross sectional area at a widest portion (132) of the vessel in a range of 1/10 to 1/25, preferably having a ratio of 1/10.

13. The system (100) according to claims 9 to 12, wherein the sample extraction device (150) is configured to mechanically couple to the settling vessel (130) and wherein a fluidically sealed connection is formed between the sample containment tube (150) and the settling vessel (130) when coupled.

14. The system 100 according to any of claims 9 to 13, wherein the control unit is configured to control and to synchronise the flow and drainage of fluids to and from the system, including to control operation of the main pump and delivery valve, air bleed valve, sample inlet and drainage valve, and the analyser.

15. A method performed by a pre-treatment settler, sample extraction and analysis system (100) according to claims 9 to 15, for automated analysis of a sample extracted from a water treatment system comprising a settling vessel, a controller, a sample extraction device, the method comprising:
actuating a delivery valve (118) of the system (100), wherein the delivery valve (118) is actuated to provide a controlled volume of fluid from the aeration tank (400) to the settling vessel;
operating an air bleed valve (161) connected to the sample extraction device (150) to prevent the release of air;
filling, via the delivery valve (118), the settling vessel (130) with a controlled volume of fluid from the aeration tank (400);
allowing the sample to settle within the settling vessel (130) and sample extraction device (150) for a settling period;
transferring, a portion of the settled sample to the inner tube portion (153) by operating the air bleed valve (161) connected to the sample extraction device (150) to affect a release of air from the inner tube portion of the sample extraction device;
extracting, via the sample inlet (156) within the inner tube portion (153) of the sample containment tube (150), a portion of the sample for analysis;
transferring, via a fluid line (157) coupled between the sample inlet (156) and an analyser (200), the portion of the sample for analysis.
